# EUROPÄISCHE PATENTSCHRIFT

(11) **EP 1 923 022 B1**
(45) Veröffentlichungstag und Bekanntmachung des Hinweises auf die Patenterteilung: **19.01.2011**
(21) Anmeldenummer: 08001262.8
(22) Anmeldetag: 08.11.2005
(51) Int. Cl.: A61F 2/42

(54) **Sprunggelenksendoprothesenelemente**
Ankle endoprosthesis elements
Eléments d'endoprothèse d'articulation de la cheville

(30) Priorität: 08.11.2004 AT 18622004
(43) Veröffentlichungstag der Anmeldung: 21.05.2008
(62) Teilanmeldung aus: 05801610.6
(73) Patentinhaber: Alphamed Medizintechnik Fischer GmbH, A-8301 Lassnitzhöhe (AT)
(72) Erfinder: Fellinger, Michael, Prof. Dr., 8010 Graz (AT); Orthner, Ernst, Prim. Doz. Dr., 4600 Wels (AT); Siorpaes, Robert, Prim. Dr., 6380 St. Johann (AT)
(74) Vertreter: Schwarz, Albin

(56) Entgegenhaltungen:
- WO-A-00/69373
- US-A- 3 889 300
- US-A- 3 975 778
- LEARDINI A ET AL: "COMPUTER-ASSISTED DESIGN OF THE SAGITTAL SHAPES OF A LIGAMENT-COMPATIBLE TOTAL ANKLE REPLACEMENT" MEDICAL AND BIOLOGICAL ENGINEERING AND COMPUTING, PETER PEREGRINUS LTD. / IFMBE, HERTS, GB, Bd. 39, Nr. 2, März 2001 (2001-03), Seiten 168-175, XP001099761 ISSN: 0140-0118

## Beschreibung

Die vorliegende Erfindung betrifft Sprunggelenksprothesenelemente, genauer eine Taluskomponente, für eine mehrteilige Sprunggelenksprothese.

Angesichts der vielen Gründe, die für eine bewegliche Lösung eines schmerzhaft destruierten oberen Sprunggelenks sprechen, war es nur folgerichtig, nach den ersten Erfolgen der Hüftund Kniegelenkendoprothetik nach entsprechenden Lösungen auch für andere Gelenke zu suchen, zumal an den direkt vom Körpergewicht belasteten Gelenken eine erfolgversprechende Alternative im Sinne einer Resektionsarthroplastik nicht zur Verfügung stand.

Bei den ersten Sprunggelenksprothesen handelte es sich um zementierte Metall-Polyethylen-Gelenke. Die bekannteste Prothese dieser Generation, die sogenannte St. Georg-Endoprothese, wies eine schlittenartige Taluskomponente, auf, die mit einer nach unten kongruent, d.h. konkav geformten Tibiakomponente artikulierte.

Die zweite Generation der nun auch zementfrei implantierbaren Endoprothesen stützte sich auf die Idee der "meniskalen" Oxford-Knieendoprothesen. Die schlittenartige Form der metallischen Taluskomponente wurde mit einer oberflächlichen Führung für einen kongruent geformten Polyethylengleitkern, der nach oben mit der planaren Unterfläche der Tibiakomponente frei beweglich artikuliert, beibehalten. Hierdurch ist der Gleitkern (Inlay) durch die entsprechend zur Talusrolle kongruent geformte Unterfläche gegen Luxationen gesichert, lässt aber nach oben hin alle Bewegungsrichtungen, u.a. im Rotationssinn und anterodorsal, zu.

Es stellte sich prinzipiell heraus, dass den mechanischen Anforderungen und somit einer hohen Langzeitüberlebensrate nur eine Sprunggelenkprothese mit einem anatomischen und biomechanisch kompatiblen Design genügen kann, die mit minimaler Knochenresektion implantiert und frei von den verbleibenden Bändern und Sehnen geführt wird. In Beachtung dieser Anforderungen sind verschiedene Typen von Sprunggelenkprothesen entwickelt und zur Behandlung eingesetzt worden.

Die heute verwendeten Endoprothesen können im Wesentlichen in ein- und multiaxiale Zweikomponentenendoprothesen sowie Dreikomponentenendoprothesen unterteilt werden. Die Dreikomponentenendoprothesen bestehen im Allgemeinen aus einer Tibia- und einer Taluskomponente sowie einem dazwischenliegenden Gleitkem.

Bekannt ist z.B. eine Dreikomponenten-Prothese (HINTEGRA^{®}; Newdeal SA, Vienne, Frankreich) mit einer anatomischen Tibia- und Taluskomponente sowie einem freibeweglichen Polyethylen(PE)-Gleitkern von ultra-hoher Dichte. Die Tibiakomponente weist eine Metallplatte mit tibialseits kleinen Verankerungspyramiden sowie einem ventralen Schild auf, welcher eine Schraubenfixation durch zwei ovale Löcher erlaubt und das Einwachsen von Narbengewebe und somit Bewegungseinschränkung verhindert. Die Taluskomponente weist eine einem Konus mit einem schmäleren medialen Seitenradius ähnliche Form auf. Ein Seitenrand medial- und lateralseits führt den PE-Gleitkern. Dieser mobile und gefühlte Gleitkern weist eine flache Oberfläche zur Tibiakomponente und eine konkave Oberfläche zur Taluskomponente auf.

Bei einer anderen bekannten Sprunggelenkendoprothese ("A.E.S- Prothese", Biomet Merck Deutschland GmbH) besteht die Tibiakomponente aus einer Metallplatte mit einem intraoperativ aufsetzbaren Kiel, während die Taluskomponente als Teil einer Zylinderoberfläche konfiguriert ist, die in Umfangsrichtung einen mittigen rinnenförmigen Einschnitt aufweist, in dem der talusseitig entsprechend geformte Inlay gleitet. Die Oberseite des Inlays ist der Tibiakomponente entsprechend planar ausgebildet.

Die Tibiakomponente einer weiteren bekannten Prothese (LINK S.T.A.R.^{®}; Waldemar Link GmbH & Co. KG) besteht aus einer Metallplatte mit hochglanzpolierter flacher Artikulationsfläche, welche zwei rückseitig angeordnete zylindrische Fixationsstege zur Verankerung im Tibiaknochen besitzt. Als talare Komponente dient eine konvexe Metallkappe, die an ihrer Unterseite in einem abgerundeten stumpfen Winkel zusammenläuft. In der Mitte der konvexen Oberfläche der Taluskomponente befindet sich, A/P-verlaufend, eine Rippe zur Führung des PE-Inlays, der dementsprechend eine konkave Oberfläche mit einer Rinne besitzt.

Eine zweiteilige und eine dreiteilige Sprunggelenksprothese sind aus der US 3,975,778 bzw. der WO 00/69373 A1 bekannt.

Die vorliegende Erfindung stellt sich die Aufgabe, Sprunggelenksprothesenelemente bzw. Sprunggelenksprothesen bereitzustellen, die eine optimale Passform des talaren Prothesenanteils bei gleichzeitiger geringer Knochenresektion besitzen. Außerdem soll ein seitliches Überstehen des Gleitkerns und somit die Entstehung eines Impingement verhindert werden.

Diese Aufgabe wird erfindungsgemäß bei einer Taluskomponente für eine mehrteilige Sprunggelenksprothese, welche Taluskomponente eine Unterseite aufweist, die nach Implantation dem knöchernen Talus zugewandt ist, dadurch gelöst, dass die dem knöchernen Talus zugewandte Unterseite als konkaves Kugelsegment ausgestaltet ist.

Es hat sich gezeigt, daß die konkave Ausgestaltung der Unterseite der Taluskomponente eine besonders schonende Präparation des Knochens ermöglicht.

In einer bevorzugten Ausführungsform ist an der als konkaves Kugelsegment ausgestalteten Unterseite ein Mittel zur Verankerung im Knochen vorgesehen.

Vorzugsweise umfasst das Mittel einen zentral angeordneten rohrförmigen Stiel und einen dazu ventral angeordneten Stift.

Gegenstand der Erfindung ist auch eine dreiteilige Sprunggelenksprothese mit einer Taluskomponente, einem Inlay und einer Tibiakomponente, welche dadurch gekennzeichnet ist, dass eine erfindungsgemäße Taluskomponente vorgesehen ist.

Ein weiterer Aspekt der Erfindung betrifft eine Tibiakomponente-Inlay-Kombination für eine mehrteilige Sprunggelenksprothese, wobei die Tibiakomponente eine Platte und ein Mittel zur Verankerung im Knochen umfasst und das Inlay eine planare tibiale Oberfläche aufweist. Erfindungsgemäß sind an der dem Inlay zugewandten Seite der Platte eine im Wesentlichen zentrale Erhebung und in der tibialen Oberfläche des Inlay für die Erhebung eine Ausnehmung vorgesehen, wobei die Ausnehmung derart gestaltet ist, dass dem Inlay gegenüber der Tibiakomponente eine Rotation sowie ein AP-Crleiten ermöglicht wird und eine seitliche Belegung des Inlay verhindert wird.

Hierdurch wird ein seitliches Überstehen des Gleitkerns und somit die Entstehung eines Impingement verhindert.

Die Erhebung kann kugelsegnentförmig ausgebildet sein und die Ausnehmung als Segment eines Zylinders mit kugelsegmentförmigen Enden ausgestaltet sein, wobei die Achse des Zylinders in A/P-Richtung ausgerichtet ist.

Vorzugsweise umfasst das Mittel zur Verankerung im Knochen einen ventral gebogenen Keil.

Nachfolgend wird die Erfindung anhand der Zeichnung näher erläutert, wobei Fig. 1 und 2 einen Querschnitt bzw. eine Ansicht einer Ausführungsform der erfindungsgemäßen Taluskomponente, Fig. 3, 4 und 5 Ansichten einer Ausführungsform der Tibiakomponente einer Tibiakomponente-Inlay-Kombination und Fig. 6, 7 und 8 eine Ansicht bzw. Querschnitte einer Ausführungsform des Inlays einer Tibiakomponente-Inlay-Kombination veranschaulichen.

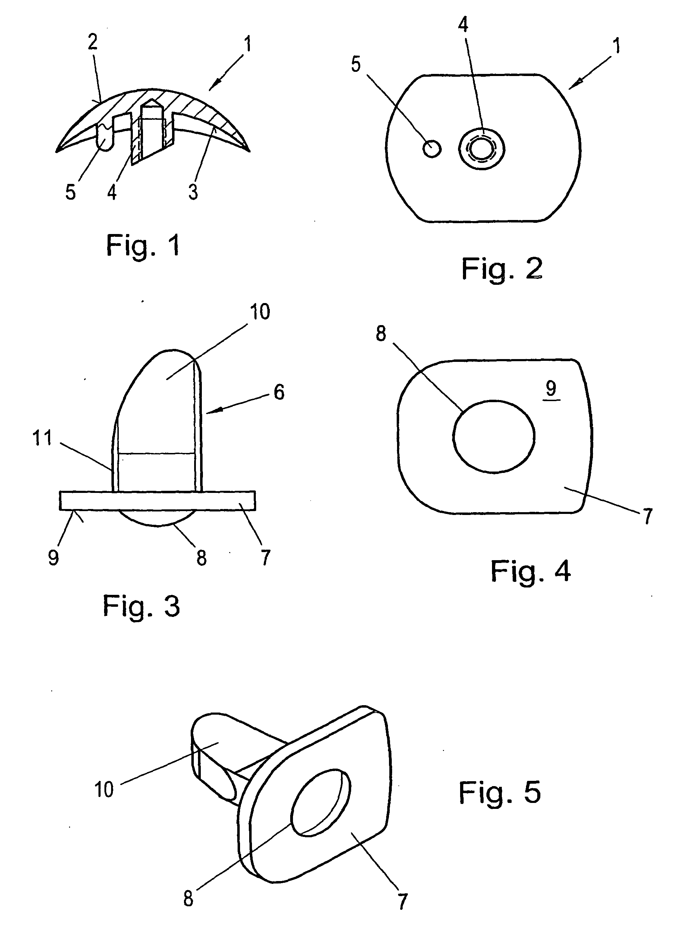

Fig. 1 zeigt einen Schnitt durch eine Ausführungsform der erfindungsgemäßen Taluskomponente 1. Dieses Element ist gewöhnlich aus biokompatiblem Metall gefertigt, beispielsweise aus CoCrMo nach ISO 5832/4. Bezugsziffer 2 bezeichnet die dem Inlay zugewandte zylindrisch konvexe Oberfläche. Mit 3 ist die als konkaves Kugelsegment ausgestaltete, dem Talus zugewandte Unterseite des Elements bezeichnet, die zum Beispiel mit Tricalciumphosphat beschichtet sein kann.
Fig. 2 zeigt eine Ansicht der Unterseite 3 der Taluskomponente 1. Im zentralen Teil der Unterseite 3 ist ein rohrförmiger Stiel 4 angeordnet, der zur Verankerung im Knochen dient und, wie dargestellt, mit einem Innengewinde versehen sein kann. Dieser ist in Richtung des zentralen Radiales der sphärischen Oberfläche ausgerichtet. Um das Einbringen des Gelenkteils zu erleichtern, ist der rohrförmige Stiel 4 abgeschrägt, und zwar derart, dass sich der längere Teil vorne befindet.

Ventral des rohrförmigen Stiels 4 ist ein Metallstift 5 angeformt. Dieser hat beispielsweise einen Durchmesser von etwa 3 mm und ist in der Darstellung parallel zur Richtung des zentralen Stiels 4 angeordnet. Der Stift 5 dient zur primären Rotationssicherung des eingebrachten talaren Prothesenelements.

In Fig. 3 ist eine Seitenansicht einer Ausführungsform der Tibiakomponente 6 einer Tibiakomponente-Inlay-Kombination dargestellt. Das tibiale Prothesenelement besteht aus einer z.B. 3 mm dicken Metallplatte 7, welche auf der Seite des Inlays eine kugelsegmentförmige Erhebung 8 trägt, welche einen Teil für die notwendige Zwangsführung des Polyinlays bildet. Die Erhebung 8 ist im Wesentlichen zentral an der Unterseite 9 der Metallplatte 7 vorgesehen, wie Fig. 4 und 5 zeigen.

Zur Vergrößerung der ossären Integrationsfläche sowie zur Rotationssicherung der Tibiakomponente 6 ist diese kranialwärts mit einem Keil 10 versehen. Dieser Keil 10 ist an seiner unteren Begrenzungsseite 11 deutlich abgeflacht, um die Insertion der Tibiakomponente 6 zu erleichtern. Der kraniale Keilanteil verjüngt sich kontinuierlich und soll damit zur Impaktierung des spongiösen Knochen beitragen.

Vorzugsweise können individuell auf die entsprechende Metallplatte 7 verschieden lange Keile 9 je nach Erfordernis aufgesetzt werden. Der längste Revisionskeil trägt dabei bevorzugt 2 Löcher für die Aufnahme von Verriegelungsbolzen. In die kugelsegmentformige Erhebung 8 sowie in die Metallplatte 7 kann ein Befestigungsmechanismus für die modularen Tibiakeile integriert werden.

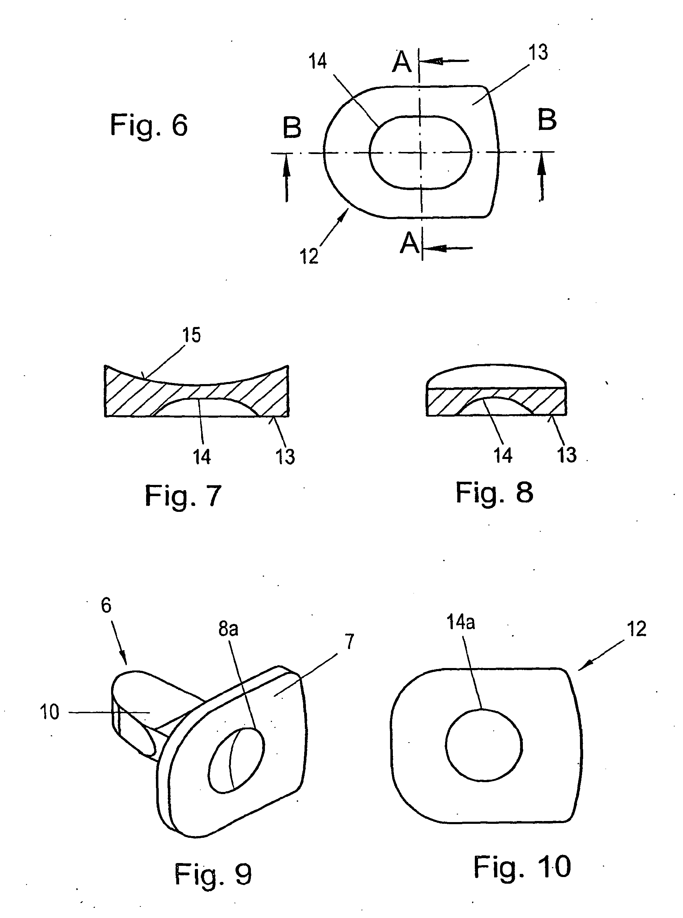

Fig. 6 zeigt eine Ansicht der tibialen Oberfläche 13 des PE-Inlays 12, der beispielsweise aus Chirulen ISO 5834 gefertigt sein kann. Die Konfiguration des Inlays 12 orientiert sich bezüglich der Form an der Metallplatte 7 der Tibiakomponente 6. Die zu verwendende Größe des Inlay 12 ist prinzipiell an der Taluskomponente angelehnt.

Die tibiale Oberfläche 13 besteht aus einer planaren Fläche, in deren Zentrum in Längsrichtung, d.h. A/R-Richtung, eine Ausnehmung 14 in Form einer Rinne vorgesehen ist, die als Zylindersegment mit kugelsegmentförmigen Enden ausgestaltet ist. Diese Form der Ausnehmung 14 lässt sich aus den in Fig. 7 und 8 dargestellten Querschnitten entlang der Linie B-B bzw. A-A der Fig. 6 erkennen. Die Ausnehmung 14 nimmt die Erhebung 8 der Tibiakomponente 6 geschlossen auf. Im Zusammenspiel ist somit das notwendige A/P-Gleiten und die Rotation des Inlays 12 gegenüber der Tibiakomponente 6 gewährleistet. Die seitliche Verschiebbarkeit gegenüber der Tibiakomponente 6 ist hingegen eingeschränkt. Dies verhindert in Bezug auf die Metallplatte 7 der Tibiakomponente 6 ein seitliches Überstehen und somit die Entstehung eines Impingements durch den Inlay 12.

Die der Taluskomponente 1 zugewandte Oberfläche 15 des Inlays 12 ist, wie in Fig. 7 dargestellt, dieser entsprechend als konkave Zylinderoberfläche ausgebildet.

## Patentansprüche

1. Taluskomponente für eine mehrteilige Sprunggelenksprothese, welche Taluskomponente (1) eine Unterseite (3) aufweist, die nach Implantation dem knöchernen Talus zugewandt ist,
**dadurch gekennzeichnet,**
**dass** die dem knöchernen Talus zugewandte Unterseite (3) als konkaves Kugelsegment ausgestaltet ist.

2. Taluskomponente nach Anspruch 1, **dadurch gekennzeichnet, dass** an der als konkaves Kugelsegment ausgestalteten Unterseite (3) ein Mittel zur Verankerung im Knochen vorgesehen ist.

3. Taluskomponente nach Anspruch 2, **dadurch gekennzeichnet, dass** das Mittel einen zentral angeordneten rohrförmigen Stiel (4) und einen dazu ventral angeordneten Stift (5) umfasst.

4. Dreiteilige Sprunggelenksprothese mit einer Taluskomponente, einem Inlay und einer Tibiakomponente, **dadurch gekennzeichnet, dass** eine Taluskomponente nach einem oder mehreren der Ansprüche 1 bis 3 vorgesehen ist.

## Claims

1. A talus component for a multipart ankle joint prosthesis, which talus component (1) has a bottom side (3) facing the osseous talus after implantation,
**characterized in that**
the bottom side (3) facing the osseous talus is configured as a concave spherical segment.

2. A talus component according to claim 1, **characterized in that** a means for anchoring in the bone is provided on the bottom side (3) configured as a concave spherical segment.

3. A talus component according to claim 2, **characterized in that** the means comprises a centrally arranged tubular shaft (4) and a pin (5) arranged ventrally thereto.

4. A three-part ankle joint prosthesis comprising a talus component, an inlay and a tibia component, **characterized in that** a talus component according to one or several of claims 1 to 3 is provided.

## Revendications

1. Composant de talus pour une prothèse d'articulation de la cheville en plusieurs parties, ledit composant de talus (1) présentant une face inférieure (3) qui, après implantation, est tournée vers le talus osseux,
**caractérisé en ce que**
la face inférieure (3) tournée vers le talus osseux est conçue comme un segment sphérique concave.

2. Composant de talus selon la revendication 1, **caractérisé en ce qu'**il est prévu un moyen d'ancrage dans l'os au niveau de la face inférieure (3) conçue comme segment sphérique concave.

3. Composant de talus selon la revendication 2, **caractérisé en ce que** le moyen comprend une barre de forme tubulaire (4) agencée au centre et une tige (5) agencée en situation ventrale par rapport à celle-ci.

4. Prothèse d'articulation de la cheville en trois pièces comprenant un composant de talus, un inlay et un composant de tibia, **caractérisée en ce qu'**il est prévu un composant de talus selon l'une ou plusieurs des revendications 1 à 3.
